# EUROPEAN PATENT APPLICATION

(11) **EP 4 040 446 A1**
(43) Date of publication of application: **10.08.2022**
(21) Application number: 22154174.1
(22) Date of filing: 31.01.2022
(51) Int. Cl.: G16H 30/20, G16H 10/60, G16H 30/40

(54) **SYSTEM AND METHOD FOR CONNECTING A MEDICAL IMAGE SYSTEM WITH AN EXTERNAL SERVICE SERVER**

(30) Priority: 03.02.2021 KR 20210015138
(71) Applicant: Seen, Dong June, Hwado-eup, Gyeonggi-do Namyangju-si (KR)
(72) Inventor: Seen, Dong June, Hwado-eup, Gyeonggi-do Namyangju-si (KR)
(74) Representative: Nederlandsch Octrooibureau

(57) **Abstract**

A connection system for connecting a medical image system to a service server can include a communication unit configured to transmit and receive files to and from each of the medical image system and the service server, where the communication unit can receive a first file written according to a first standard from the medical image system and receive a second file written according to a second standard from the service server; a storage unit that stores the attributes of the first file; a conversion unit that generates a third file by converting the second file into the first standard, where the conversion unit can generate the third file such that at least one attribute of the third file corresponds to the attributes of the first file stored in the storage unit; and a control unit that controls the communication unit, the storage unit, and the conversion unit.

## Description

### CROSS-REFERENCE TO RELATED APPLICATIONS

This application claims the benefit of Korean Patent Application No. 10-2021-0015138, filed with the Korean Intellectual Property Office on February 3, 2021, the disclosure of which is incorporated herein by reference in its entirety.

### BACKGROUND

### 1. Technical Field

The invention relates to a system and method for connecting a medical image system, more particularly to a system and method for connecting a medical image system to an external service server in a manner that increases compatibility with external remote services and thus increases the utility of the medical image system.

### 2. Description of the Related Art

Developments in imaging technology have seen results in the field of medicine, and imaging is currently being utilized in many branches of the medical field. Many hospitals and clinical facilities have imaging or scanning equipment installed as a means of diagnosis, where a medical image system is used to manage the images obtained from such equipment. A typical example of a medical image system is the PACS (picture archiving and communication system), where the PACS is a system that cost-efficiently stores images obtained from various imaging equipment and allows convenient access. A PACS can receive images from scanning equipment such as, for example, an X-ray scanner, a CT scanner, an MRI scanner, and the like.

In the field of medicine, an error occurring in an image resource or, in fact, any type of information associated with an image resource can lead to a fatal result for a patient, and as such, regulations pertaining to medical scanning equipment and medical image systems are much stricter compared to other fields.

For example, data used in medical image systems and scanning equipment handling medical images is required to follow a single unified standard specialized for the medical field, and in many countries, the DICOM standard is used as this standard. The DICOM (digital imaging and communications in medicine) standard was developed for managing and transmitting medical images and makes it possible for different hospitals and clinics to commonly use the numerous types of imaging and scanning equipment manufactured by different companies. To prevent errors resulting from medical image files being mapped to incorrect information, a file following the DICOM standard can have information regarding the patient, date, etc., stored as attributes within the file itself.

As another example of more stringent regulations applied on medical devices, regulations in the Republic of Korea require that medical image systems receive government certification after manufacture, and a medical image system that has received certification cannot be altered in any way whatsoever.

Regulations such as the above may limit the utility of medical images, since external remote services for interpreting medical image files may not necessarily use files following the DICOM standard. That is, files that are managed in a medical image system after being obtained from medical scanning equipment may be transferred to an external remote service for professional interpretation, AI interpretation, etc., where such external service can provide the interpretation results in the form of text files, picture files such as JPG files, etc., or files based on other standards. Since the existing medical image system is manufactured to only be capable of managing DICOM files, it may be difficult to save the interpretation results again in the medical image system, even if the results are from interpreting one or more files stored in the medical system.

Thus, the medical image system suffers from a very low compatibility with external services, since the medical image system cannot be modified once it receives government certification after its manufacture. While one may consider the method of manufacturing the medical image system such that it is capable of managing files of various standards from the start, there is a limit to identifying all of the standards currently used by external services, and it is impossible to predict new standards that will be commonly used in the future. Moreover, upgrading the existing medical image systems currently already installed in most hospitals and clinics and receiving certification again would require excessive amounts of cost and effort. Thus, there is a need for a system and method for connecting a medical image system with external service servers that increases the compatibility of the medical image system with external remote services to thereby increase the utility of the medical image system.

### SUMMARY OF THE INVENTION

An aspect of the invention, which was conceived to resolve the problem described above, is to provide a system and method for connecting a medical image system to an external service server in a manner that can increase compatibility with the external remote services and can thus increase the utility of the medical image system.

Other objectives of the invention will be more clearly understood from the embodiments set forth below.

One aspect of the invention provides a connection system for connecting a medical image system to a service server. The connection system can include a communication unit configured to transmit and receive files to and from each of the medical image system and the service server, where the communication unit can receive a first file written in the format of a first standard from the medical image system and receive a second file written in the format of a second standard from the service server; a storage unit configured to store at least one attribute of the first file; a conversion unit configured to generate a third file by converting the second file into the format of the first standard, where the conversion unit can generate the third file such that at least one attribute of the third file corresponds to the at least one attribute of the first file stored in the storage unit; and a control unit configured to control the communication unit, the storage unit, and the conversion unit.

A connection system according to an embodiment of the invention can include one or more of the following features. For example, the connection system can be connected to a multiple number of service servers, and the control unit can select one of the multiple service servers for transmitting the first file based on an attribute of the first file or based on an outside command.

The medical image system can include a storage device that is configured to store a multiple number of files and further store a first database, which may store at least one attribute of the stored files, and the storage unit of the connection system can store a second database that corresponds to the first database of the medical image system. In this case, the control unit can add at least one attribute of the third file in the second database after the third file is generated or after the third file is transmitted to the medical image system. Also, in cases where the connection system is connected to a multiple number of service servers, the control unit can select one of the multiple service servers for transmitting the first file based on at least one attribute of the first file recorded in the second database.

Before the first file is transmitted to the service server, the conversion unit can convert the first file into the format of a standard different from the first standard.

Another aspect of the invention provides a connection method performed at a connection system connected to a medical image system and to a service server. The connection method can include: receiving a first file written in the format of a first standard from the medical image system; transmitting the first file to the service server; receiving a second file written in the format of a second standard different from the first standard from the service server; generating a third file by converting the second file into in the format of the first standard such that at least one attribute of the third file corresponds to an attribute of the first file; and transmitting the third file to the medical image system.

In cases where the medical image system includes a storage device that stores a multiple number of files and a first database storing the attributes of the stored files and the connection system includes a storage unit that stores a second database corresponding to the first database of the medical image system, a connection method according to an embodiment of the invention can further include adding at least one attribute of the third file to the second database after the generating of the third file.

An embodiment of the invention having one or more of the above features can provide a system and method for connecting a medical image system with an external service server in a manner that increases compatibility with external remote services and to thereby increase the utility of the medical image system.

### BRIEF DESCRIPTION OF THE DRAWINGS

FIG. 1 is a diagram conceptually illustrating the operation of a medical image system.
FIG. 2 is a diagram conceptually illustrating the operation of a connection system according to an embodiment of the invention.
FIG. 3 is a block diagram illustrating the composition of a connection system according to an embodiment of the invention.
FIG. 4 is a diagram conceptually illustrating the relationships between medical image systems, service servers, and a connection system according to an embodiment of the invention.
FIG. 5 is a flow diagram illustrating a method for connecting a medical image system with a service server according to an embodiment of the invention.

### DETAILED DESCRIPTION OF THE INVENTION

As the invention allows for various changes and numerous embodiments, particular embodiments will be illustrated in the drawings and described in detail in the written description. However, this is not intended to limit the invention to particular modes of practice, and it is to be appreciated that all changes, equivalents, and substitutes that do not depart from the spirit and technical scope of the invention are encompassed by the invention. In the description of the invention, certain detailed explanations of the related art are omitted if it is deemed that they may unnecessarily obscure the essence of the invention.

The terms used in the present specification are merely used to describe particular embodiments and are not intended to limit the invention. An expression used in the singular encompasses the expression of the plural, unless it has a clearly different meaning in the context. In the present specification, it is to be understood that terms such as "including" or "having," etc., are intended to indicate the existence of the features, numbers, steps, actions, components, parts, or combinations thereof disclosed in the specification and are not intended to preclude the possibility that one or more other features, numbers, steps, actions, components, parts, or combinations thereof may exist or may be added.

While such terms as "first" and "second," etc., can be used to describe various components, such components are not to be limited by the above terms. The above terms are used only to distinguish one component from another.

Certain embodiments of the invention will be described below in more detail with reference to the accompanying drawings. Those components that are the same or are in correspondence are rendered the same reference numeral, and redundant descriptions are omitted.

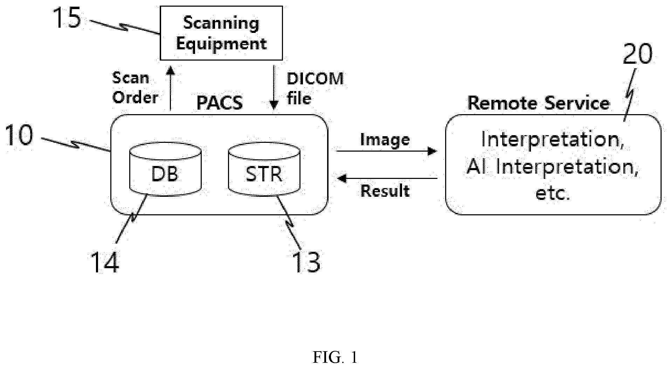

FIG. 1 is a diagram conceptually illustrating the operation of a medical image system.

Referring to FIG. 1, the medical staff of a hospital or clinic, when requiring scanning such as for an X-ray image, etc., may input a scan order through a medical information system such as an OCS, EMR, etc. The transferred scan order can include the order number, patient information, scan information (e.g., scan site, scan method), etc.

A medical image system 10 may transfer the scan order to a scanning equipment 15 such as an X-ray scanning device, etc., and the scanning equipment 15 may obtain a required image in correspondence to the received order. Here, the image obtained by the scanning equipment 15 may generally be generated in the format of the DICOM standard.

The DICOM-format file thus generated may be transferred back to the medical image system 10 and stored at an appropriate path. To interpret an image stored in the medical image system 10, the image can be transferred to a service server 20 that provides an external remote service, and the service server 20 can transmit the interpretation results back to the medical image system 10. While there are external services that provide the interpretation results as files of the DICOM format, there are also services that provide the interpretation results or relevant additional information as files of a text format, files of the JPG format, etc., and many existing medical image systems 10 are designed to be incapable of managing files written according to a standard other than the DICOM standard.

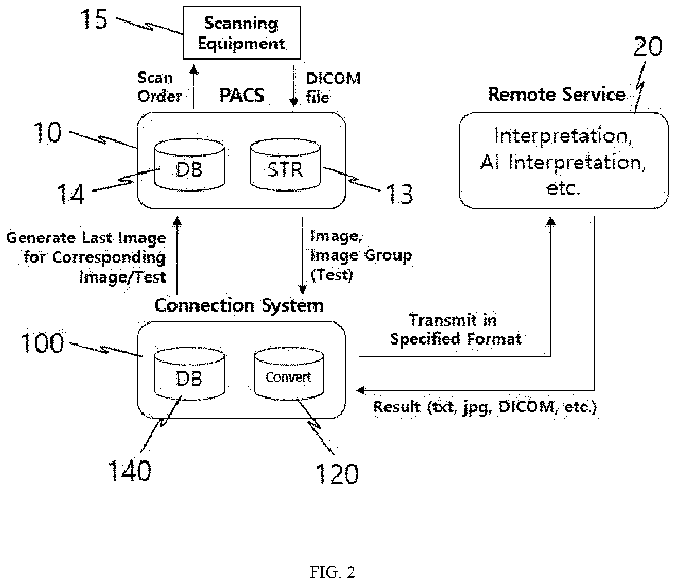

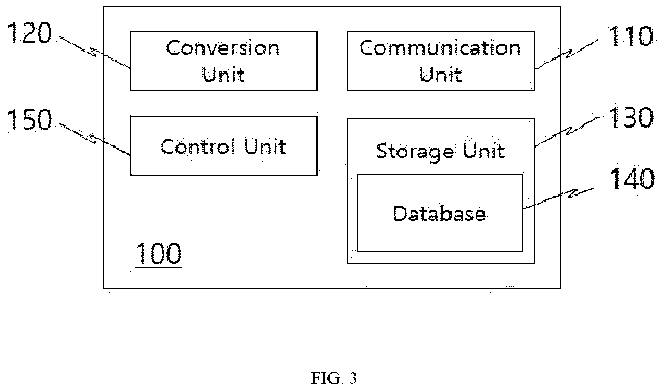

FIG. 2 is a diagram conceptually illustrating the operation of a connection system according to an embodiment of the invention, and FIG. 3 is a block diagram illustrating the composition of a connection system according to an embodiment of the invention.

As illustrated in FIG. 2, a connection system 100 according to an embodiment of the invention can be a system for connecting a medical image system 10, such as a PACS, etc., with a service server 20 that provides an external remote service.

At the medical image system 10 side, an image file obtained by the scanning equipment 15 can be stored in the storage device 13 of the medical image system 10 in the form of a file written in the format of a first standard, while information related to the file stored in the storage device 13, the attributes of the file, or the like can be stored in a database 14. While the drawings depict the storage device 13 and the database 14 as separate components, it should be apparent that the database 14 and the image files are all stored in a memory and that the portions where the database 14 and the image files are stored can be collectively referred to as a single storage device 13.

From among the files stored in the medical image system 10, the files requiring an external service can be transmitted to the connection system 100, and the connection system 100 can transfer these to the service server 20 providing an external service. Similarly, the results provided by the service server 20 can be transmitted to the connection system 100, and the connection system 100 can be transferred to the medical image system 10. In this embodiment, the external service can be a service related to image interpretation, and the file transmitted from the medical image system 10 can be an image file. Of course, the invention is not limited thus, and different files can be transmitted and received for a variety of external services.

Referring to FIG. 2 and FIG. 3, the connection system 100 can mainly include a communication unit 110, a conversion unit 120, a storage unit 130, and a control unit 150.

The communication unit 110 can be configured to transmit and receive files to and from each of the medical image system 10 and the service server 20. The communication unit 110 can be configured to exchange files written in the format of a first standard when communicating with the medical image system 10 and exchange files written in formats of a variety of standards when communicating with the service server 20. Here, the first standard for example can be the DICOM standard, for example, but the invention is not limited thus.

As mentioned above, the medical image system 10 can transmit one or more files to the connection system 100, where the files can be received through the communication unit 110. For the sake of convenience, the one or more files that require an external service and are transmitted from the medical image system 10 to the connection system 100 are referred to herein as first files. A first file may be generated according to a first standard such as DICOM, etc. The first file can be a single image file or can include multiple files. For example, a series of image files scanned in a single examination session can be received simultaneously.

The storage unit 130 can store the attributes of the first file. The storing of the attributes can be performed during a process of identifying the and recording the attributes of the first file after the first file is received or during a process of storing the attributes of the files stored in the medical image system 10 beforehand. For example, the storage unit 130 can maintain its own database 140, where the database 140 can be updated to be the same as or in correspondence to the database 14 of the medical image system 10. Such updates can be performed periodically, whenever an entry is added to the database 14 of the medical image system 10, or in a manner corresponding to a combination of the two.

The communication unit 110 can transmit the received first file to the service server 20, and the service server 20 can perform a service such as interpretation, etc., on the first file, to generate a service result file based on the first file. For the sake of convenience, the files that are generated based on the first files are referred to herein as second files. A second file can be written in the format of a standard different the first standard, i.e., in the format of a second standard. Depending on what the circumstances may require, the first file transferred from the connection system 100 to the service server 20 can be kept in the format of the first standard or can be converted into the format of the second standard or another third standard before its transmission.

The conversion unit 120 can convert the second file received from the service server 20 into a format of the first standard. For the sake of convenience, the result of converting a second file into the format of the first standard in this manner is referred to herein as a third file. That is, the third file and the second file can be files that relate to the same content but are written in formats of different standards. Generally, however, a DICOM file can typically include more attributes, including patient information, date information, etc., to prevent matching errors. Therefore, the conversion unit 120 can refer to the attributes of the first file stored in the storage unit 130 and generate the third file such that at least one of the attributes of the third file corresponds to the attributes of the first file stored in the storage unit 130.

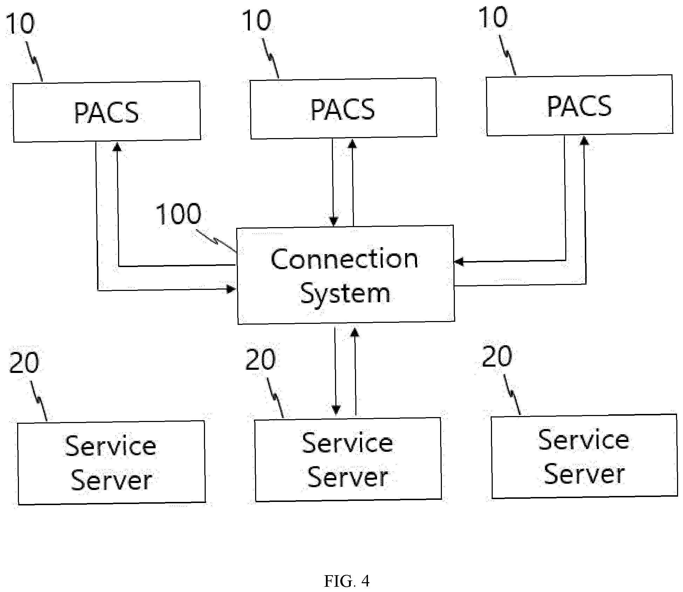

For example, the files stored in the storage device 13 of the medical image system 10 can be written in the format of the first standard, and information such as patient information, scan site, scan date, image type, etc., can be stored by using the attributes of the files themselves and/or the storage location. For instance, the medical image system 10 can manage the scan date of a particular file by storing the file in a folder representing a particular date, within a folder representing a particular month, within a folder representing a particular year. Thus, in the present specification, mention of an attribute of a file is intended to encompass not only the attributes of the file itself but also the storage path of the file. Therefore, in cases where a connection system 100 according to an embodiment of the invention is connected to multiple medical image systems 10 as in the illustration of FIG. 4, information regarding which medical image system 10 a first file was received from can be regarded as being encompassed within the concept of the attributes of the file.

The attributes of a first file received from the medical image system 10 can be stored in the storage unit 130, and when necessary, the attributes of the first filed can be conferred to the third file when the third file is generated. As described above, the point at which the attributes of the first file are stored in the storage unit 130 can be after the first file is received from the medical image system 10 or can be at an earlier time when the database 140 is maintained to be in correspondence with the database 14 of the medical image system 10.

For example, supposing a case in which three first files are generated from a scan for a particular site of a particular patient, the attributes representing information on the patient, site, and scan date can be the same for all of the three first files, and different serial numbers (e.g., #1, #2, and #3) can be assigned to differentiate the three files. If the first files are transferred to the service server 20 and two second files are generated as a result of the external service, the second files may not include information regarding the patient, site, and scan date. In such a case, the conversion unit 120 can convert the two second files into the format of the first standard to generate two third files, where the conversion unit 120 can reference the attributes of the first files stored in the storage unit 130 to confer the same attributes representing information regarding the patient, site, and scan date to each of the two third files and can also assign new serial numbers (e.g., #4 and #5) to differentiate the two files.

The communication unit 110 can transmit the third files generated at the conversion unit 120 back to the medical image system 10. Since the third files were generated in the format of the first standard, which is manageable by the medical image system 10, the third files can be freely utilized at the medical image system 10.

In cases where the storage unit 130 maintains a database 140 in correspondence to the database 14 of the medical image system 10, information related to the third files transmitted to the medical image system 10, i.e., the attributes of the third files, can be added to the database 140. The point at which the attributes of a third file are added to the database 140 can be at any time after the third file is generated, for example, even after the third file is transmitted to the medical image system 10.

The control unit 150 can control the operations of the communication unit 110, conversion unit 120, and storage unit 130 overall. For example, the control unit 150 can control, among others, the process for storing the attributes of the first file in the storage unit 130 and the process by which the conversion unit 120 generates a third file.

A connection system 100 according to an embodiment of the invention can be connected to a multiple number of service servers 20, as illustrated in FIG. 4. In this case, the control unit 150 can select the service server 20 to which the first file is to be transmitted, from among the multiple service servers 20. The control unit 150 can select a service server 20 based on the attributes of the first file, for example determining the most suitable service server 20 based on attributes such as the scan site, image type, etc. It is also possible to have an index designating a particular service server 20 included among the attributes of a file. Of course, in cases where a first file is received according to a separate outside command, such as a request from the medical image system 10 itself or from a particular service server 20, it is also possible to select the service server 20 based on the corresponding outside command.

In certain embodiments, the process by which the control unit 150 selects the service server 20 for transmitting the first file from among the multiple number of service servers 20 can entail referencing the database 140. For example, when a first file is received through the communication unit 110, the control unit 150 can use certain attributes of the first file to search for records related to the first file within the database 140 and can use the information matching the search to determine the service server 20 to which the first file should be transmitted.

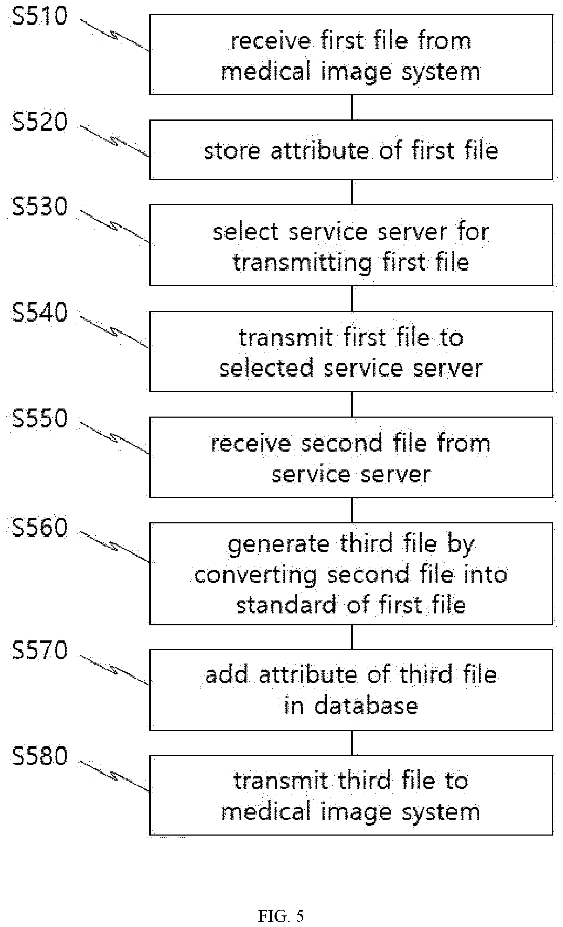

FIG. 5 is a flow diagram illustrating a method for connecting a medical image system with a service server according to an embodiment of the invention. This connection method may be performed by a connection system 100 that is connected to one or more medical image systems 10 and one or more service servers 20 as described above.

First, the connection system 100 can receive one or more first file, written in the format of a first standard, from a medical image system 10 (operation S510). As described above, the first standard can be the DICOM standard, but the invention is not limited thus.

The connection system 100 can store the attributes of the received first file (operation S520). For example, the connection system 100 can store the attributes of the received first file in a particular storage unit 130 after the first file is received. In certain other embodiments, the storing of the attributes of the first file can be performed before the receiving (operation S510) of the first file. For example, a database 140 corresponding to the database 14 maintained at the medical image system 10 can be maintained at the storage unit 130 of the connection system 100.

The connection system 100 can select the service server 20 to which the received first file will be transmitted (operation S530). The connection 100 can make the decision based on one or more attributes of the first file or based on an outside command. In cases where the connection system 100 is connected to just one service server 20, the selecting of a service server 20 (operation S530) can be omitted. For example, while it may be necessary to select a required service server 20 if a connection system 100 is installed close to the terminal of a medical image system 10 and is connected over a network such as the Internet, etc., to a multiple number of service servers 20, it may not be necessary to select a service server 20 if a connection system 100 is installed close to the terminal of a particular service server 20 and is connected over a network such as the Internet, etc., to a multiple number of medical image systems 10. In certain embodiments, a connection system 100 can be connected to multiple medical image systems 10 and multiple service servers 20, as illustrated in FIG. 4, in which case an operation of selecting a service server 20 (operation S530) can be required.

The connection system 100 can transmit the first file to the selected service server 20 (operation S540). The connection system 100 can transmit the first file such that the first file maintains the format of the first standard or can transmit the first file after converting the first file into the format of a standard other than the first standard as necessary.

The service server 20 can perform a service such as interpretation, etc., on the first file and transmit a second file, which may be written in the format of a second standard that is different from the first standard, as a result of the service, whereupon the connection system 100 can receive the second file (operation S550).

Then, the connection system 100 can generate a third file (operation S560) by converting the second file into the format of the first standard, which is the standard of the format of the first file. During this process, the connection system 100 can generate the third file such that at least one of the attributes of the third file corresponds to the stored attributes of the first file as necessary.

In cases where the connection system 100 maintains a database 140 that corresponds to the database 14 of the medical image system 10, the connection system 100 can add a record of the third file, including the attributes of the third file, in the database 140 (operation S570). Of course, this operation may not require a separate procedure; for instance, the record of the third file can be added automatically at a later time when the database 140 of the connection system 100 is updated to be synchronized with the database 14 of the medical image system 10.

The connection system 100 can transmit the third file, which has been converted to the format of the first standard, back to the medical image system 10 (operation S580). In certain embodiments of the invention, one or more attributes of the third file, such as its storage path, etc., can be written to correspond to the attributes of the first file, so that the third file may be stored grouped together with the original first file at the medical image system 10.

While the foregoing provides a description with reference to an embodiment of the invention, it should be appreciated that a person having ordinary skill in the relevant field of art would be able to make various modifications and alterations to the invention without departing from the spirit and scope of the invention set forth in the scope of claims below.

## Claims

1. A connection system connected to a medical image system and to a service server, the connection system comprising:
a communication unit configured to transmit and receive files to and from each of the medical image system and the service server, the communication unit configured to receive a first file written in a format of a first standard from the medical image system and configured to receive a second file written in a format of a second standard from the service server;
a storage unit configured to store at least one attribute of the first file;
a conversion unit configured to generate a third file by converting the second file into the format of the first standard, the conversion unit configured to generate the third file such that at least one attribute of the third file corresponds to the at least one attribute of the first file stored in the storage unit; and
a control unit configured to control the communication unit, the storage unit, and the conversion unit,
wherein the medical image system includes a storage device configured to store a plurality of files including the first file, the storage device further stores a first database, the first database storing at least one attribute of the stored plurality of files,
the storage unit of the connection system stores a second database corresponding to the first database of the medical image system, and the control unit adds at least one attribute of the third file in the second database after the third file is generated or after the third file is transmitted to the medical image system.

2. The connection system of claim 1, wherein the connection system is connected to a plurality of service servers, and
the control unit selects one of the plurality of service servers for transmitting the first file based on at least one attribute of the first file recorded in the second database or based on an outside command.

3. The connection system of claim 1, wherein the conversion unit converts the first file into a format of a standard different from the first standard before the first file is transmitted to the service server.

4. A connection method performed at a connection system connected to a medical image system and to a service server, the connection method comprising:
storing records of a first database in a second database, the first database stored in a storage device of the medical image system and storing at least one attribute of a plurality of files;
receiving a first file among the plurality of files from the medical image system, the first file written in a format of a first standard;
transmitting the first file to the service server;
receiving a second file from the service server, the second file written in a format of a second standard different from the first standard;
generating a third file by converting the second file into in the format of the first standard such that at least one attribute of the third file corresponds to a stored attribute of the first file;
adding at least one attribute of the third file in the second database; and
transmitting the third file to the medical image system.
